**Europäisches Patentamt**

(19) **European Patent Office**

**Office européen des brevets**

(11) Numéro de publication : **0 207 831 B1**

(12) ## FASCICULE DE BREVET EUROPEEN

(45) Date de publication du fascicule du brevet :
**27.02.91 Bulletin 91/09**

(51) Int. Cl.$^5$ : **C07D 519/04**

(21) Numéro de dépôt : **86401179.6**

(22) Date de dépôt : **03.06.86**

(54) **Procédé de préparation de vincristine.**

(30) Priorité : **03.06.85 FR 8508333**

(43) Date de publication de la demande :
**07.01.87 Bulletin 87/02**

(45) Mention de la délivrance du brevet :
**27.02.91 Bulletin 91/09**

(84) Etats contractants désignés :
**AT BE CH DE FR GB IT LI LU NL SE**

(56) Documents cités :
**EP-A- 0 079 785**

(73) Titulaire : **PIERRE FABRE MEDICAMENT**
**125, Rue de la Faisanderie**
**F-75116 Paris (FR)**

(72) Inventeur : **Tarroux, Roger**
**122, rue Théron Périé**
**F-81100 Castres (FR)**
Inventeur : **Fahy, Jacques**
**11, avenue d'Hauterive**
**F-81290 Labruguiere (FR)**
Inventeur : **Hatinguais, Philippe**
**Le Landou, Chemin de Bel Air**
**F-81100 Castres (FR)**

(74) Mandataire : **Ahner, Francis et al**
**CABINET REGIMBEAU, 26, avenue Kléber**
**F-75116 Paris (FR)**

## Description

La présente invention concerne un procédé particulièrement efficace de préparation de la vincristine.

Les alcaloïdes cytostatiques du Vinca rosea L. Catharanthus roseus G.Don) sont des anticancéreux maintenant bien connus et particulièrement utiles. Compte-tenu de la faible quantité de vincristine présente dans le Catharanthus, un assez grand nombre de moyens de préparation ont été proposés par les chimistes. C'est ainsi que FR-A-2 296 418 décrit la synthèse de la vincristine par couplage de la catharanthine et de la vindoline. D'autres laboratoires ont obtenu la transformation de la vinblastine en vincristine par oxydation dans des conditions contrôlées, très strictes.

FR-A-2 210 393 et US-A-3 899 493 réalisent l'oxydation par l'acide chromique à –30, –90°C dans un mélange acétone-acide acétique ou dans le chloroforme-acide acétique à –55°C.

Dans US-A 4 375 432, on utilise également un dérivé chromique, en milieu acide à –65°C, –50°C dans un milieu solvant à base de THF. En outre, EP-A-37 289 revendique une oxydation par un mélange sel ferreux, eau oxygénée, perchlorate dans l'acétonitrile. ZA-A-82 08939 décrit un procédé avec l'acide chromique et un mélange éther-chloroforme.

HU-A-23 638 propose le diterbutylchromate dans l'acide pélargonique, et enfin EP-A- 117 861 obtient la transformation vinblastine/ vincristine en oxydant par le permanganate de potassium en milieu acide acétique. Il est clair que ces alcaloïdes dimères constituent une matière précieuse du fait de leur faible quantité dans les matières premières végétales, et par conséquent que les procédés de synthèse ou d'hémisynthèse performants sont d'un intérêt extrême.

Dans cette optique, nous avons réalisé une étude approfondie de l'oxydation, dans des solvants variés compatibles avec les solubilités des réactifs et une basse température.

Il est curieux de constater la grande importance du solvant mis en oeuvre, la majorité d'entre eux ne permettant d'ailleurs pas d'accéder correctement à la vincristine : le méthanol, le diméthyl formamide, le chloroforme, le toluène, le benzène, le monoéthyléther de l'éthylène glycol, le diméthyléther de l'éthylèneglycol, le diméthylsulfoxyde donnent des rendements pratiquement nuls. L'acétate de méthyl, l'acétonitrile, le dioxanne, l'acétate de butyle, le formiate d'éthyle, l'acétone, la méthylisobutylcétone, la méthyléthylcétone donnent des rendements médiocres. Nous avons confirmé les bons rendements obtenus dans le THF (US-A-4 375 432), mais nous avons constaté des difficultés de séparation dues aux émulsions stables au moment de l'extraction du milieu réactionnel par le chlorure de méthylène.

De façon tout à fait surprenante, nous avons découvert que l'acétate d'éthyle constituant un excellent solvant permettant l'oxydation de la vinblastine en vincristine, comparativement à tous ceux déjà préconisés dans la technique antérieure.

L'agent oxydant mis en oeuvre est l'acide chromique ou ses sels, et la réaction est conduite à une température comprise entre –83°C et –25°C.

Le procédé d'hémisynthèse de la vincristine ainsi mis en oeuvre selon l'invention permet d'obtenir les avantages suivants :
* rendement élevé (80-90%)
* non toxicité (comparé à l'acide formique, les solvants chlorés ou aromatiques, l'acétonitrile)
* ne forme pas de peroxydes (comparé à l'éther, THF)
* relativement peu inflammable (comparé à l'éther, THF)
* permet une grande échelle de température, à partir de –83°C (comparé à l'acide acétique, l'acétonitrile ou l'acide pélargonique), et
* facilite la séparation finale de la vincristine après alcalinisation du fait de la non miscibilité à l'eau (comparé à l'acide acétique, l'anhydride acétique, l'acétone, l'acide formique, le THF, l'acétonitrile).

## Exemple

20 g de vinblastine base sont solubilisés dans 2 litres d'acétate d'éthyle et 40 ml d'acide acétique. La température est amenée à –70, –60°C et l'on ajoute lentement sous agitation énergique une solution de 30 g de bichromate de sodium dans 130 ml d'eau et 8 ml d'acide sulfurique, en maintenant la température du milieu réactionnel à –70, –60°C.

L'agitation est maintenue 2 heures.

200 ml d'ammoniaque à 10% sont ajoutés lentement de façon à neutraliser le milieu réactionnel sans échauffement excessif.

L'acétate d'éthyle contenant la vincristine base brute est décanté. Une deuxième extraction est réalisée avec 1 litre du même solvant. Les phases organiques réunies sont lavées et évaporées sous vide à 40-45°C.

Le résidu de vincristine brute est solubilisé dans 100 ml de toluène et la solution est purifiée par chromatographie sur colonne de silice Merck® 60 mesh avec un gradient d'élution toluène-méthanol (3 à 20% de méthanol).

La vincristine est repurifiée par cristallisation dans le méthanol, puis le sulfate ou autre sel est obtenu suivant les procédés classiques de salification avec un acide therapeutiquement acceptable.

Rendement : 70-80% en moles.

## Revendications

1. Procédé d'hémisynthèse de la vincristine caractérisé en ce que l'on oxyde la vinblastine ou ses sels par l'acide chromique ou ses sels à basse température dans l'acétate d'éthyle additionné d'acide acétique et en ce que la vincristine base, libérée par adjonction d'ammoniaque dans le milieu réactionnel, est obtenue directement par décantation.

2. Procédé selon la revendication 1, caractérisé par le fait que la réaction d'oxydation est effectuée entre −83°C et −25°C.

3. Procédé selon l'une des revendications 1 et 2, caractérisé par le fait que la vincristine brute est purifiée par chromatographie sur silice avec mise en oeuvre d'un gradient d'élution toluène-méthanol.

4. Procédé selon l'une des revendications 1 à 3, caractérisé par le fait que la vincristine est purifiée par cristallisation dans le méthanol.

5. Procédé selon l'une des revendications 1 à 4, caractérisé en ce que la vincristine est salifiée avec un acide thérapeutiquement acceptable.

## Ansprüche

1. Verfahren zur Halbsynthese von Vincristin, dadurch gekennzeichnet, daß man Vinblastin oder seine Salze mit Chromsäure oder ihren Salzen bei niedriger Temperatur in mit Essigsäure versetztem Ethylacetat oxidiert und daß man die durch Zusatz von Ammoniak zu dem Reaktionsmedium freigesetzte Vincristinbase direkt durch Dekantieren gewinnt.

2. Verfahren nach Anspruch 1, dadurch gekennzeichnet, daß die Oxidationsreaktion bei −83°C bis −25°C durchgeführt wird.

3. Verfahren nach einem der Ansprüche 1 und 2, dadurch gekennzeichnet, daß das rohe Vincristin durch Chromatographie an Siliciumdioxid unter Anwendung einer Gradienten-Elution mit Toluol-Methanol, gereinigt wird.

4. Verfahren nach einem der Ansprüche 1 bis 3, dadurch gekennzeichnet, daß das Vincristin durch Kristallisation in Methanol gereinigt wird.

5. Verfahren nach einem der Ansprüche 1 bis 4, dadurch gekennzeichnet, daß das Vincristin mit einer therapeutisch brauchbaren Säure in ein Salz übergeführt wird.

## Claims

1. A method of hemisyntheis of vineristine, characterised in that vinblastine or salts thereof are oxidized by chromic acid or salts thereof at a low temperature in ethyl acetate also containing acetic acid, and the vincristine base is liberated by adding ammonia to the reaction medium and obtained directly by decantation.

2. A method according to claim 1, characterised in that the oxidation reaction is brought about between −83°C and −25°C.

3. A method according to claim 1 or 2, characterised in that the crude vincristine is purified by chromatography on silica, using a toluene-methanol elution gradient.

4. A method according to any of claims 1 to 3, characterised in that vincristine is purified by crystallization from methanol.

5. A method according to any of claims 1 to 4, characterised in that vincristine is converted into a salt, using a therapeutically acceptable acid.